# EUROPÄISCHE PATENTSCHRIFT

(11) **EP 2 563 762 B1**
(45) Veröffentlichungstag und Bekanntmachung des Hinweises auf die Patenterteilung: **22.07.2015**
(21) Anmeldenummer: 11717250.2
(22) Anmeldetag: 26.04.2011
(51) Int. Cl.: C07D 213/64

(54) **VERFAHREN ZUR HERSTELLUNG SUBSTITUIERTER PYRIDIN- 2 - ONE**
METHOD FOR PRODUCING SUBSTITUTED PYRIDIN-2-ONE
PROCÉDÉ POUR PRODUIRE DES PYRIDIN- 2 - ONES SUBSTITUÉES

(30) Priorität: 29.04.2010 DE 102010028362
(43) Veröffentlichungstag der Anmeldung: 06.03.2013
(73) Patentinhaber: Bayer Intellectual Property GmbH, 40789 Monheim (DE)
(72) Erfinder: KRÜGER, Joachim, 40489 Düsseldorf (DE); GROSSBACH, Danja, 42329 Wuppertal (DE); PAULSEN, Holger, 40724 Hilden (DE); KROH, Walter, 42115 Wuppertal (DE)
(74) Vertreter: BIP Patents
(86) Internationale Anmeldenummer: PCT/EP2011/056588
(87) Internationale Veröffentlichungsnummer: WO 2011/134963

(56) Entgegenhaltungen:
- WO-A1-2008/155032
- WO-A1-2008/155033
- US-A- 4 789 745
- US-A1- 2002 019 531
- SAU FAN YIP: "Room-Temperature Copper-Catalyzed alpha-Arylation of Malonates", ORGANIC LETTERS, Bd. 9, Nr. 17, 21. Juni 2007 (2007-06-21), Seiten 3469-3472, XP000002656654, in der Anmeldung erwähnt

## Beschreibung

Die vorliegende Erfindung betrifft ein Verfahren zur Herstellung von substituierten 3-(2-Hydroxyethyl)-1-[4-nitrophenyl]pyridin-2(1H)-onen, die als wichtige Zwischenverbindungen zur Herstellung von Arzneimitteln dienen.

Die Verbindung 5-Chlor-N-{[(5S)-3-{4-[3-{2-[(trans-4-hydroxycyclohexyl)-amino]ethyl}-2-oxopyridin-1(2H)-yl]-3,5-dimethylphenyl}-2-oxo-1,3-oxazolidin-5-yl]methyl}thiophen-2-carboxamid ist aus WO 2008/155032 bekannt und entspricht der Formel (Ia) 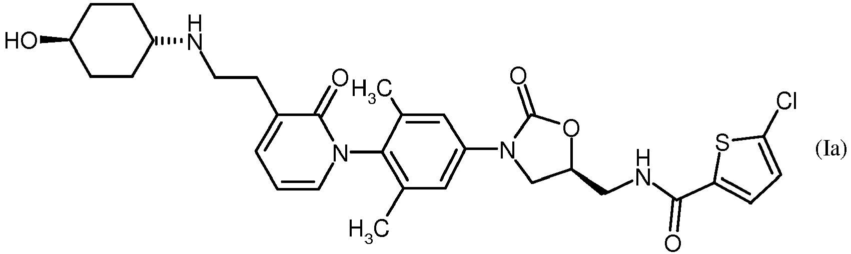 und die Verbindung 5-Chlor-N-[((5S)-3-{4-[3-(2-hydroxyethyl)-2-oxopyridin-1(2H)-yl]-3-methylphenyl}-2-oxo-1,3-oxazolidin-5-yl)methyl]thiophen-2-carboxamid ist aus WO 2008/155033 bekannt und entspricht der Formel (IIb)

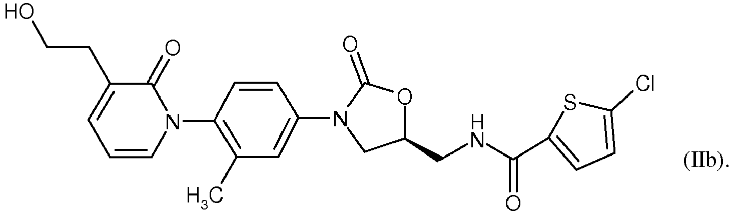

Bei den Verbindungen der Formel (Ia) und (IIb) handelt es sich um duale Inhibitoren der Blutgerinnungsfaktoren Xa und Thrombin (Faktor IIa), die insbesondere als Antikoagulantien wirken. Die Verbindungen hemmen sowohl Thrombin als auch Faktor Xa und verhindern durch Hemmung der Thrombinproduktion und -aktivität auf Gerinnseln deren potentielles Wachstum.

In WO 2008/155032 ist auch eine Methode zur Herstellung der Verbindungen der Formel (Ia) und (IIb) beschrieben:

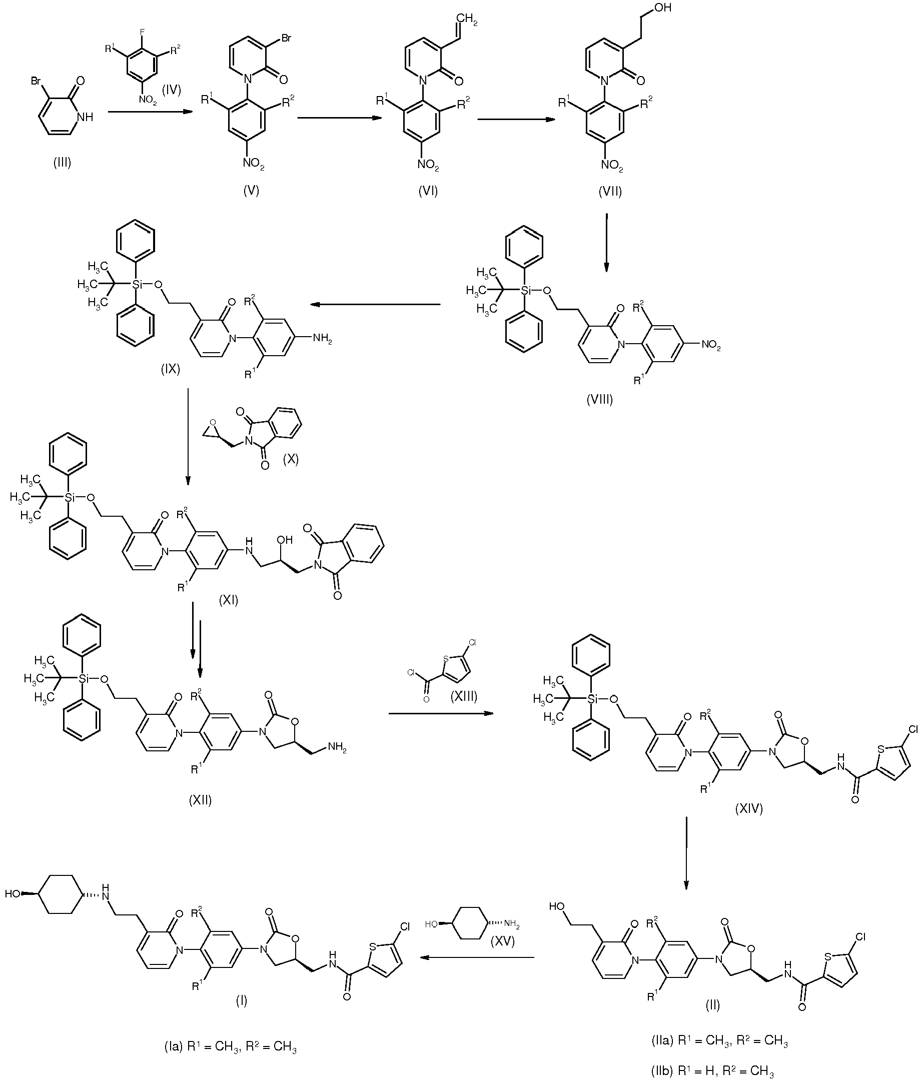

Zur Herstellung der Verbindung der Formel (Ia) wird 3-Brompyridin-2(1*H*)-on (III) mit 1-Fluor-2,5-dimethyl-4-nitrobenzol (IV) zu 3-Brom-1-(2,6-dimethyl-4-nitrophenyl)pyridin-2(1H)-on (V) umgesetzt. Anschließend wird (V) mit Tributylvinyl-zinn und Tetrakis(triphenylphosphin)palladium in 1-(2,6-Dimethyl-4-nitrophenyl)-3-vinylpyridin-2(1H)-on (VI) überführt. Durch Hydroborierung und Oxidation von (VI) gelangt man zu 1-(2,6-Dimethyl-4-nitrophenyl)-3-(2-hydroxyethyl)pyridin-2(1H)-on (VII). Um die Hydroxygruppe zu schützen, wird (VII) mit tert-Butyl(chlor)diphenylsilan in Gegenwart einer Base umgesetzt, und man erhält 3-(2-{[tert-Butyl(diphenyl)silyl]oxy}ethyl)-1-(2,6-dimethyl-4-nitrophenyl)pyridin-2(1H)-on (VIII). Die Reduktion der Nitrogruppe in (VIII) führt zu 1-(4-Amino-2,6-dimethylphenyl)-3-(2-{[tert-butyl(diphenyl)silyl]oxy}ethyl)pyridin-2(1H)-on (IX). Es folgt die Umsetzung von (IX) mit (S)-Epoxyphthalimid (X) zu 2-[(2R)-3-({4-[3-(2-{[tert-Butyl(diphenyl)silyl]oxy}ethyl)-2-oxopyridin-1(2H)-yl]-3,5-dimethyl-phenyl}amino)-2-hydroxy-propyl]-1H-isoindol-1,3(2H)-dion (XI). Ringschluss zum Oxazolidinon und die Abspaltung der Phthalimidgruppe liefert ausgehend von (XI) 1-{4-[(5S)-5-(Aminomethyl)-2-oxo-1,3-oxazolidin-3-yl]-2,6-dimethylphenyl}-3-(2-{[tert-butyl-(diphenyl)silyl]oxy}ethyl)pyridin-2(1H)-on (XII), das mit 5-Chlorthiophen-2-carbonylchlorid (XIII) zu N-[((SS)-3-{4-[3-(2-{[tert-Butyl-(diphenyl)silyl]oxy}ethyl)-2-oxopyridin-1(2H)-yl]-3,5-dimethyl-phenyl}-2-oxo-1,3-oxazolidin-5-yl)methyl]-5-chlorthiophen-2-carboxamid (XIV) umgesetzt wird. Abspaltung der Schutzgruppe in (XIV) liefert 5-Chlor-N-[((5S)-3-{4-[3-(2-hydroxyethyl)-2-oxopyridin-1(2H)-yl]-3,5-dimethyl-phenyl}-2-oxo-1,3-oxazolidin-5-yl)methyl]thiophen-2-carboxamid (IIa), das mit trans-4-Aminohexanol (XV) zu 5-Chlor-N-{[(5S)-3-{4-[3-{2-[(trans-4-hydroxycyclohexyl)-amino]ethyl}-2-oxopyridin-1(2H)-yl]-3,5-dimethylphenyl}-2-oxo-1,3-oxazolidin-5-yl]methyl}thiophen-2-carboxamid (Ia) umgesetzt wird.

Dieses aus WO 2008/155032 bekannte Verfahren weist aber verschiedene Nachteile in der Reaktionsführung auf, die sich besonders ungünstig bei der Herstellung der Verbindung der Formel (I) und (II) in technischem Maßstab auswirken. Als besonders nachteilig erweist sich die in WO 2008/155032 beschriebene Synthese in den Syntheseschritten (V) zu (VI) (Stille-Kupplung) und (VI) zu (VII) (Hydroborierung).

So erlaubt eine Durchführung in technischem Maßstab keine toxischen Reagenzien. Dies ist *per se* von Nachteil, darüber hinaus müssen diese toxischen Substanzen aus dem Endprodukt (I) bzw. (II) bis unterhalb der jeweils im Produkt aus regulatorischen Gründen zulässigen Höchstgrenze entfernt werden, was einen zusätzlichen Aufwand bedeutet. Weiterhin sollten die Reagenzien einfach und preisgünstig zugänglich sein.

Daraus ergibt sich die Aufgabe der vorliegenden Erfindung, ein vereinfachtes Verfahren zur Herstellung der Verbindungen der Formeln (I bzw. Ia) und (II bzw. IIb) in technischem Maßstab unter Vermeidung toxischer Reagenzien bereitzustellen.

Kwong et al., Org. Lett., 2007, 9, 3469-3472 beschreibt die Umsetzung von Iodaromaten und Malonsäurediethylester mit Kupferiodid in Gegenwart von 2-Pyridincarbonsäure und Cäsiumcarbonat bei Raumtemperatur in Dioxan. Cäsiumcarbonat wird von Kwong et al. im Überschuß von 3.0 Äquivalenten eingesetzt und trägt neben einem hohen Preis auch deutlich zur Salzfracht bei.

Überraschenderweise wurde nun gefunden, dass sich durch Modifikation bestimmter Reaktionsparameter bei der aus WO 2008/155032 bekannten Synthese die Verbindungen der Formeln (I bzw. Ia) und (II bzw. IIb) auch in größeren Mengen in guter Ausbeute und Reinheit herstellen lassen. Dazu wurde die Stille-Kupplung in Syntheseschritt (V) zu (VI) durch eine Kupfer katalysierte Malonsäurester Addition, Verseifung und Decarboxylierung ersetzt und die Hydroborierung in Syntheseschritt (VI) zu (VII) durch eine einfache Reduktion mit Boran-Komplex durchgeführt. Diese Syntheseschritte erlauben ein effizientes scale-up der Synthese, bei der damit kostengünstigere, gut verfügbare und weniger toxische Reagenzien verwendet werden.

Gegenstand der vorliegenden Erfindung ist ein Verfahren zur Herstellung von Verbindungen der Formel 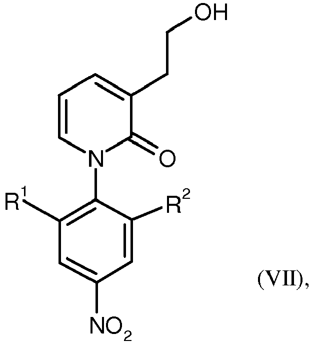 in welcher
R¹ für Wasserstoff oder C₁-C₃-Alkyl steht
   und
R² für C₁-C₃-Alkyl steht,
   oder
R¹ für Wasserstoff steht
   und
R² für C₁-C₃-Alkoxy oder C₁-C₃-Alkoxymethyl steht,
dadurch gekennzeichnet, dass die Carboxyl-Gruppe in Verbindungen der Formel 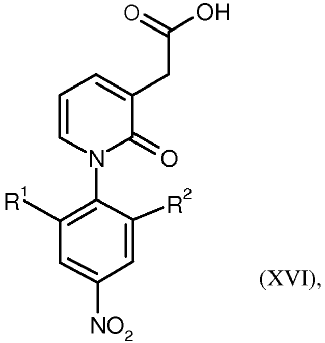 in welcher
R¹ für Wasserstoff oder C₁-C₃-Alkyl steht
   und
R² für C₁-C₃-Alkyl steht,
   oder
R¹ für Wasserstoff steht
   und
R² für C₁-C₃-Alkoxy oder C₁-C₃-Alkoxymethyl steht,
in Gegenwart eines Boran-Komplexes reduziert wird.

Bevorzugt ist ein Verfahren zur Herstellung von Verbindungen der Formel (VII), in welcher R¹ für Wasserstoff oder Methyl steht und R² für Methyl steht, oder R¹ für Wasserstoff steht und R² für Methoxy oder Methoxymethyl steht, dadurch gekennzeichnet, dass die Carboxyl-Gruppe in Verbindungen der Formel (XVI), in welcher R¹ für Wasserstoff oder Methyl steht und R² für Methyl steht, oder R¹ für Wasserstoff steht und R² für Methoxy oder Methoxymethyl steht, in Gegenwart eines Boran-Komplexes reduziert wird.

Ganz besonders bevorzugt ist ein Verfahren zur Herstellung von Verbindungen der Formel (VII), in welcher R¹ für Methyl steht und R² für Methyl steht, dadurch gekennzeichnet, dass die Carboxyl-Gruppe in Verbindungen der Formel (XVI), in welcher R¹ für Methyl steht und R² für Methyl steht, in Gegenwart eines Boran-Komplexes reduziert wird.

Die Reduktion der Carboxyl-Gruppe in Gegenwart eines Boran-Komplexes erfolgt in einem Lösungsmittel, bevorzugt in einem Temperaturbereich von 0°C bis zum Rückfluss des Lösungsmittels bei Normaldruck.

Lösungsmittel sind beispielsweise Ether wie Dioxan, Tetrahydrofuran, 2-Methyl-tetrahydrofuran, 1,2-Dimethoxyethan oder Methyl-tert-butylether, oder andere Lösungsmittel wie Dichlormethan oder Toluol, bevorzugt ist 2-Methyl-tetrahydrofuran.

Boran-Komplexe sind beispielsweise Boran-Dimethylsulfid, Boran-Tetrahydrofuran, Boran-Diethylanilin oder Catecholboran, bevorzugt ist Boran-Diethylanilin.

Der Boran-Komplex wird im Überschuss eingesetzt, bevorzugt werden 1 bis 5 Äquivalente Boran-Komplex verwendet, besonders bevorzugt werden 2 Äquivalente Boran-Komplex verwendet.

In einer bevorzugten Ausführungsform der vorliegenden Erfindung beträgt die Reaktionstemperatur 0°C bis 50°C, besonders bevorzugt beträgt die Reaktionstemperatur 15°C bis 25°C.

Bei der Durchführung des erfindungsgemäßen Verfahrens wird das Diethylanilin vorzugsweise durch Extraktion aus der durch Salzsäure sauer gestellten wässrigen Phase abgetrennt, nachdem Natriumchlorid zugegeben wurde. Die Extraktion erfolgt bevorzugt mit Essigsäureethylester, Dichlormethan oder Toluol.

Auf diese Weise kann die in WO 2008/155032 und WO 2008/155033 verwendete Hydroborierung vermieden werden. Hydroborierungen werden in der Regel in hoher Verdünnung durchgeführt und die Reagenzien sind teuer. Der anschließende Oxidationsschritt ist im technischen Maßstab aufwändig, da hohe Sicherheitsstandards eingehalten werden müssen.

Gegenstand der vorliegenden Erfindung ist auch ein Verfahren zur Herstellung von Verbindungen der Formel 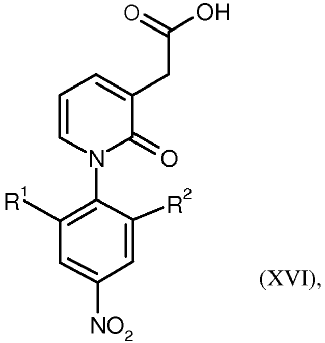 in welcher
R¹ für Wasserstoff oder C₁-C₃-Alkyl steht
   und
R² für C₁-C₃-Alkyl steht,
   oder
R¹ für Wasserstoff steht
   und
R² für C₁-C₃-Alkoxy oder C₁-C₃-Alkoxymethyl steht,
dadurch gekennzeichnet, dass Verbindungen der Formel 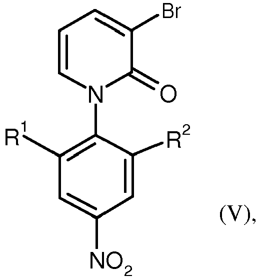 in welcher
R¹ für Wasserstoff oder C₁-C₃-Alkyl steht
   und
R² für C₁-C₃-Alkyl steht,
   oder
R¹ für Wasserstoff steht
   und
R² für C₁-C₃-Alkoxy oder C₁-C₃-Alkoxymethyl steht,
in einem Eintopfverfahren zunächst in Gegenwart eines Kupferkatalysators mit einem Malonsäureester und einer Base und anschließend mit einer Base und Wasser umgesetzt werden.

Bevorzugt ist ein Verfahren zur Herstellung von Verbindungen der Formel (XVI), in welcher R¹ für Wasserstoff oder Methyl steht und R² für Methyl steht, oder R¹ für Wasserstoff steht und R² für Methoxy oder Methoxymethyl steht, dadurch gekennzeichnet, dass Verbindungen der Formel (V), in welcher R¹ für Wasserstoff oder Methyl steht und R² für Methyl steht, oder R¹ für Wasserstoff steht und R² für Methoxy oder Methoxymethyl steht, in einem Eintopfverfahren zunächst in Gegenwart eines Kupferkatalysators mit einem Malonsäureester und anschließend mit einer Base umgesetzt werden.

Ganz besonders bevorzugt ist ein Verfahren zur Herstellung von Verbindungen der Formel (XVI), in welcher R¹ für Methyl steht und R² für Methyl steht, dadurch gekennzeichnet, dass Verbindungen der Formel (V), in welcher R¹ für Methyl steht und R² für Methyl steht, in einem Eintopfverfahren zunächst in Gegenwart eines Kupferkatalysators mit einem Malonsäureester und anschließend mit einer Base umgesetzt werden.

Der erste Schritt der Umsetzung mit einem Malonsäureester in Gegenwart eines Kupferkatalysators und einer Base erfolgt in einem Lösungsmittel, bevorzugt in einem Temperaturbereich von 15°C bis zum Rückfluss des Lösungsmittels bei Normaldruck.

Lösungsmittel sind beispielsweise Dioxan, Tetrahydrofuran und 2-Methyl-tetrahydrofuran, bevorzugt ist Dioxan.

Kupferkatalysatoren sind beispielsweise ein vorab hergestellter Bis-(2-Pyridincarboxylato)Kupfer(II)-Komplex oder dessen verschiedene Hydratformen, oder aus Kupferiodid, Kupferchlorid, Kupfer(II)bromid, Kupfer(II)triflat oder Kupfer(II)sulfat, mit 2-Pyridincarbonsäure in situ hergestellte Kupferkatalysatoren, bevorzugt ist vorab hergestellter Bis-(2-Pyridin-carboxylato)Kupfer(II)-Komplex.

Malonsäureester sind beispielsweise Diethylmalonsäureester, Dibenzylmalonsäureester, Di-tert-butylmalonsäureester oder Kalium-ethylmalonsäureester, bevorzugt ist Diethylmalonsäureester.

Basen sind beispielsweise Natrium- oder Kalium-tert.-butylat, Kalium-, Natrium- oder Cäsiumcarbonat, Cäsiumfluorid oder Natriumhydrid, bevorzugt ist Natrium- oder Kalium-tert.-butylat, besonders bevorzugt ist Natrium-tert.-butylat.

In einer bevorzugten Ausführungsform der vorliegenden Erfindung wird der Malonsäureester in einem vorgelagerten Schritt mit der Base deprotoniert und anschließend wird die Verbindung der Formel (V) und der Katalysator zu dem Reaktionsgemisch dazugegeben.

In einer bevorzugten Ausführungsform der vorliegenden Erfindung wird der Malonsäureester in dem vorgelagerten Schritt der Deprotonierung zu einer Suspension von Kalium-tert.-butylat in Dioxan bei einer Temperatur von 80°C bis 100°C getropft, bevorzugt ist eine Temperatur von 90°C.

In einer bevorzugten Ausführungsform der vorliegenden Erfindung wird bei der Verwendung von Kalium-tert.-butylat als Base die Verbindung der Formel (V) in einer Konzentration von 0.1 bis 0.4 molar verwendet, bevorzugt ist eine Konzentration von 0.15 bis 0.3 molar.

In einer bevorzugten Ausführungsform der vorliegenden Erfindung wird bei der Verwendung von Natrium-tert.-butylat als Base die Verbindung der Formel (V) in einer Konzentration von 0.2 bis 0.6 molar verwendet, bevorzugt ist eine Konzentration von 0.25 bis 0.4 molar.

In einer bevorzugten Ausführungsform der vorliegenden Erfindung wird der Kupferkatalysator getrennt hergestellt und als Kupferkomplex zur Reaktion gegeben. Dazu wird Kupfer(II)chlorid mit 2-Pyridincarbonsäure in einem Aceton/Ethanol-Gemisch und unter Zusatz von einer 0.1%igen Harnstofflösung umgesetzt (Synthesis and Reactivity in Inorganic, Metal-Organic, and Nano-Metal Chemistry, 35(9), 695-702; 2005*).* Aus dem Reaktionsgemisch fällt quantitativ der gewünschte Kupferkomplex (Bis-(2-Pyridincarboxylato)Kupfer(II)-Komplex) kristallin an, der unter Stickstoff abgesaugt und mit Aceton gewaschen wird.

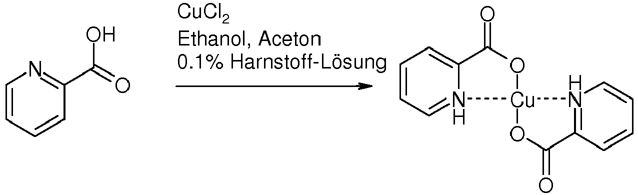

Überraschenderweise wurde bei der Herstellung von [1-(2-Methyl-4-nitrophenyl)-2-oxo-1,2-dihydropyridin-3-yl]-essigsäure festgestellt, dass dieser vorab hergestellte Bis-(2-Pyridin-carboxylato)Kupfer(II)-Komplex um Größenordnungen aktiver als der in-situ hergestellte Katalysator aus Kupferiodid und 2-Pyridincarbonsäure ist. Beispielsweise benötigt man bei einem Kalium-tert.-butylat vermittelten Prozess mit in-situ Herstellung des Katalysators 20 mol% Kupferiodid und 40 mol% 2-Pyridincarbonsäure, damit sich die Ausgangsverbindung innerhalb von 8 h vollständig zum Produkt umsetzt. Fährt man denselben Ansatz hingegen mit 10 mol% des getrennt hergestellten Bis-(2-Pyridin-carboxylato)Kupfer(II)-Komplexes, so ist die Reaktion nach 4 h unter sonst identischen Bedingungen vollständig. Noch drastischer zeigt sich dieser Effekt bei den Natrium-tert.-butylat vermittelten Reaktionen. Der Einsatz von 20 mol% Kupferiodid und 40 mol% 2-Pyridincarbonsäure zur in-situ Herstellung des Katalysators führt zu einem Umsatz von lediglich 48% nach 8 Stunden, während 7.5 mol% des Bis-(2-Pyridin-carboxylato)Kupfer(II)-Komplexes einen Umsatz von 99% innerhalb von 3.5 h liefert.

In einer bevorzugten Ausführungsform der vorliegenden Erfindung werden 3.0 bis 4.0 Äquivalente an Diethylmalonsäureester verwendet, bevorzugt sind 3.5 Äquivalente an Diethylmalonsäureester.

Der Vorteil dieses Verfahrens gegenüber dem von Kwong et al., Org. Lett., 2007, 9, 3469-3472 beschriebenen Verfahren liegt darin, dass der Kupferkatalysator getrennt herstellt und dem Reaktionsgemisch in kristalliner Form zugefügt wird. Die Reaktion mit diesem Katalysator verläuft viel schneller und mit einer geringeren Menge an Katalysator. Außerdem kann auf Cäsiumcarbonat verzichtet werden, das teuer ist und eine große Salzfracht mitbringt.

Der zweite Schritt der Umsetzung mit einer Base erfolgt in einem Lösungsmittel in Kombination mit Wasser, bevorzugt in einem Temperaturbereich von 15°C bis 40°C bei Normaldruck.

Lösungsmittel sind beispielsweise Dioxan-Wasser oder Tetrahydrofuran-Wasser, bevorzugt ist Dioxan-Wasser.

Basen sind beispielsweise Alkalihydroxide wie Lithium-, Natrium- oder Kaliumhydroxid, bevorzugt ist Natriumhydroxid.

In einer bevorzugten Ausführungsform der vorliegenden Erfindung wird das erhaltene Reaktionsgemisch nach der kupferkatalysierten Umsetzung mit einem Malonsäureester in der ersten Stufe aufkonzentriert und die unlöslichen Bestandteile werden abfiltriert, bevor das Reaktionsgemisch in der zweiten Stufe mit einer Base umgesetzt wird.

In einer bevorzugten Ausführungsform der vorliegenden Erfindung erfolgt die Aufreinigung der Verbindung der Formel (XVI) durch Waschen der wässrigen Lösung des Natriumsalzes der als Zwischenprodukt entstehenden Dimalonsäure, das anschließende Ansäuern der wässrigen Lösung, um die Decarboxylierung zu ermöglichen, und das Extrahieren der Verbindung der Formel (XVI) aus der wässrigen Lösung mit Methylenchlorid.

In einer bevorzugten Ausführungsform der vorliegenden Erfindung wird das erhaltene Rohprodukt der Verbindung der Formel (XVI) in einem anschließenden Schritt zur weiteren Reinigung aus einem Gemisch aus Dioxan und Methyl-tert-butylether umkristallisiert.

Die Herstellung der Verbindungen der Formeln (I) bis (V) und (VIII) bis (XV) erfolgt, wie bereits auch in WO 2008/155032 und WO 2008/155033 offenbart.

Die Umsetzung von Verbindungen der Formel (V) zu Verbindungen der Formel (XVI) kann auch in zwei Stufen erfolgen, so dass zunächst Verbindungen der Formel (V) in Gegenwart eines Kupferkatalysators mit einem Malonsäureester und einer Base umgesetzt werden und die Reaktionsmischung zu Verbindungen der Formel 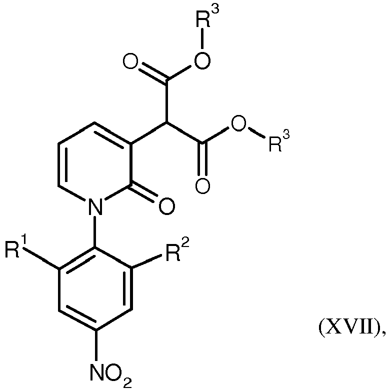 in welcher
R¹ für Wasserstoff oder C₁-C₃-Alkyl steht
   und
R² für C₁-C₃-Alkyl steht,
   oder
R¹ für Wasserstoff steht
   und
R² für C₁-C₃-Alkoxy oder C₁-C₃-Alkoxymethyl steht, und
R³ für Ethyl, tert-Butyl oder Benzyl steht,
umgesetzt und aufgearbeitet wird, und in der zweiten Stufe Verbindungen der Formel (XVII) mit einer Base und Wasser zu Verbindungen der Formel (XVI) umgesetzt werden.

Die Umsetzung der ersten Stufe erfolgt wie die Umsetzung des ersten Schrittes des Eintopfverfahren und die Umsetzung der zweite Stufe erfolgt wie die Umsetzung des zweiten Schrittes des Eintopfverfahren.

### Syntheseschema:

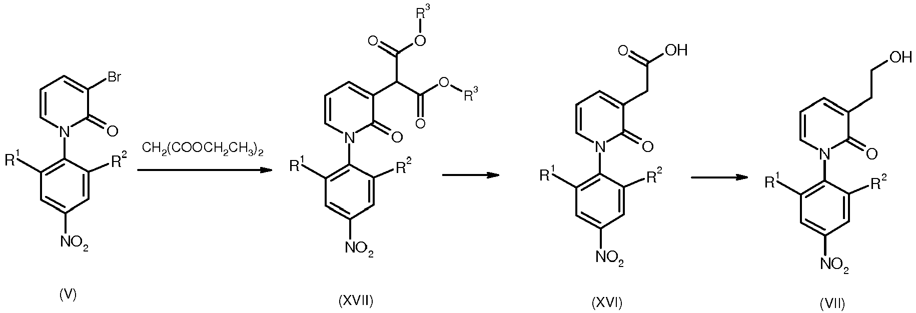

Gegenstand der vorliegenden Erfindung sind Verbindungen der Formel 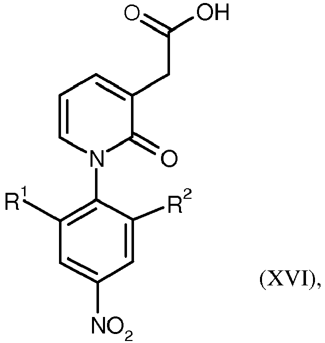 in welcher
R¹ für Wasserstoff oder C₁-C₃-Alkyl steht
   und
R² für C₁-C₃-Alkyl steht,
   oder
R¹ für Wasserstoff steht
   und
R² für C₁-C₃-Alkoxy oder C₁-C₃-Alkoxymethyl steht,
und ihre Salze, ihre Solvate und die Solvate ihrer Salze.

Gegenstand der vorliegenden Erfindung sind auch Verbindungen der Formel (XVI), in welcher R¹ für Wasserstoff oder Methyl steht und R² für Methyl steht, oder R¹ für Wasserstoff steht und R² für Methoxy oder Methoxymethyl steht.

Gegenstand der vorliegenden Erfindung sind auch Verbindungen der Formel (XVI), in welcher R¹ für Methyl steht und R² für Methyl steht.

Erfindungsgemäße Verbindungen sind die Verbindungen der Formel (XVI) und deren Salze, Solvate und Solvate der Salze; die von Formel (XVI) umfassten Verbindungen der nachfolgend genannten Formeln und deren Salze, Solvate und Solvate der Salze sowie die von Formel (XVI) umfassten, nachfolgend als Ausführungsbeispiele genannten Verbindungen und deren Salze, Solvate und Solvate der Salze, soweit es sich bei den von Formel (XVI) umfassten, nachfolgend genannten Verbindungen nicht bereits um Salze, Solvate und Solvate der Salze handelt.

Die erfindungsgemäßen Verbindungen können in Abhängigkeit von ihrer Struktur in stereoisomeren Formen (Enantiomere, Diastereomere) existieren. Die Erfindung umfasst deshalb die Enantiomeren oder Diastereomeren und ihre jeweiligen Mischungen. Aus solchen Mischungen von Enantiomeren und/oder Diastereomeren lassen sich die stereoisomer einheitlichen Bestandteile in bekannter Weise isolieren.

Sofern die erfindungsgemäßen Verbindungen in tautomeren Formen vorkommen können, umfasst die vorliegenden Erfindung sämtliche tautomere Formen.

Als Salze sind im Rahmen der vorliegenden Erfindung physiologisch unbedenkliche Salze der erfindungsgemäßen Verbindungen bevorzugt. Umfasst sind aber auch Salze, die für pharmazeutische Anwendungen selbst nicht geeignet sind aber beispielsweise für die Isolierung oder Reinigung der erfindungsgemäßen Verbindungen verwendet werden können.

Physiologisch unbedenkliche Salze der erfindungsgemäßen Verbindungen umfassen Säureadditionssalze von Mineralsäuren, Carbonsäuren und Sulfonsäuren, z.B. Salze der Chlorwasserstoffsäure, Bromwasserstoffsäure, Schwefelsäure, Phosphorsäure, Methansulfonsäure, Ethansulfonsäure, Toluolsulfonsäure, Benzolsulfonsäure, Naphthalindisulfonsäure, Essigsäure, Trifluoressigsäure, Propionsäure, Milchsäure, Weinsäure, Äpfelsäure, Zitronensäure, Fumarsäure, Maleinsäure und Benzoesäure.

Physiologisch unbedenkliche Salze der erfindungsgemäßen Verbindungen umfassen auch Salze üblicher Basen, wie beispielhaft und vorzugsweise Alkalimetallsalze (z.B. Natrium- und Kaliumsalze), Erdalkalisalze (z.B. Calcium- und Magnesiumsalze) und Ammoniumsalze, abgeleitet von Ammoniak oder organischen Aminen mit 1 bis 16 C-Atomen, wie beispielhaft und vorzugsweise Ethylamin, Diethylamin, Triethylamin, Ethyldiisopropylamin, Monoethanolamin, Diethanolamin, Triethanolamin, Dicyclohexylamin, Dimethylaminoethanol, Prokain, Dibenzylamin, N-Methylmorpholin, Arginin, Lysin, Ethylendiamin, *N*-Methylpiperidin und Cholin.

Als Solvate werden im Rahmen der Erfindung solche Formen der erfindungsgemäßen Verbindungen bezeichnet, welche in festem oder flüssigem Zustand durch Koordination mit Lösungsmittelmolekülen einen Komplex bilden. Hydrate sind eine spezielle Form der Solvate, bei denen die Koordination mit Wasser erfolgt.

Die Erfindung wird nachstehend durch bevorzugte Ausführungsbeispiele näher erläutert, auf welche sie jedoch nicht eingeschränkt ist. Soweit nicht anders angegeben, beziehen sich alle Mengenangaben auf Gewichtsprozente.

### Ausführungsbeispiele

### Abkürzungen

- CDI: Carbonyldiimidazol
- d: Dublett (bei NMR)
- DC: Dünnschicht-Chromatographie
- DCI: direkte chemische Ionisation (bei MS)
- dd: Doppeltes Dublett (bei NMR)
- DMAP: 4-Dimethylaminopyridin
- DMF: *N,N*-Dimethylformamid
- DMSO: Dimethylsulfoxid
- d: Tag(e)
- d. Th.: der Theorie (bei Ausbeute)
- eq.: Äquivalent(e)
- ESI: Elektrospray-Ionisation (bei MS)
- h: Stunde(n)
- HPLC: Hochdruck-, Hochleistungsflüssigchromatographie
- LC-MS: Flüssigchromatographie-gekoppelte Massenspektroskopie
- m: Multiplett (bei NMR)
- min: Minute(n)
- MS: Massenspektroskopie
- NMP: N-Methylpyrrolidon
- NMR: Kernresonanzspektroskopie
- RP: reverse phase (bei HPLC)
- RT: Raumtemperatur
- Rₜ: Retentionszeit (bei HPLC)
- s: Singulett (bei NMR)
- THF: Tetrahydrofuran

NMR-Spektroskopie: Alle Spektren wurden kalibriert gegen Tetramethylsilan als internen Standard (5 = 0).

### HPLC-, LC-MS- und GC-MS-Methoden:

Methode 1 (HPLC): Säule Zorbax SB-Aq 3 mm x 150 mm, 3.5 µm; Temperatur: 45°C; Eluent A: wässriger Puffer bestehend aus 1.36 g/L Kaliumdihydrogenphosphat und 1.15 g/L Phosphorsäure (85%ig); Eluent B: Acetonitril; Gradient: 0-20 min 95% A auf 20% A, 20-25 min 20% A; Fluß: 0.5 mL/min; UV-Detektion bei 210 nM.

Methode 2 (HPLC): Säule Zorbax SB-Aq 3 mm x 150 mm, 3.5 µm; Temperatur: 45°C; Eluent A: wässriger Puffer bestehend aus 1.36 g/L Kaliumdihydrogenphosphat und 0.68 mL Phosphorsäure (85%ig)/L Wasser; Eluent B: Acetonitril; Gradient: 0-3 min 80% A auf 45% A, 3-13 min 45% A auf 20% A, 13-25 min 20% A; Fluß: 0.5 mL/min; UV-Detektion bei 220 nM.

Methode 3 (HPLC): Instrument: HP 1100 mit DAD-Detektion; Säule: Kromasil 100 RP-18, 60 mm x 2.1 mm, 3.5 µm; Eluent A: 5 ml Perchlorsäure (70%ig)/L Wasser, Eluent B: Acetonitril; Gradient: 0 min 2% B, 0.5 min 2% B, 4.5 min 90% B, 6.5 min 90% B, 6.7 min 2% B, 7.5 min 2% B; Fluß: 0.75 mL/min; Temperatur: 30°C; UV-Detektion bei 210 nm.

Methode 4 (LC-MS): Instrument: Waters ACQUITY SQD UPLC System; Säule: Waters Acquity UPLC HSS T3 1.8 µ 50 mm x 1 mm; Eluent A: 1 L Wasser + 0.25 mL 99%ige Ameisensäure, Eluent B: 1 L Acetonitril + 0.25 mL 99%ige Ameisensäure; Gradient: 0.0 min 90% A → 1.2 min 5% A → 2.0 min 5% A; Ofen: 50°C; Fluss: 0.40 mL/min; UV-Detektion: 210 - 400 nm.

Methode 5 (LC-MS): Instrument: Micromass Quattro Premier mit Waters UPLC Acquity; Säule: Thermo Hypersil GOLD 1.9µ 50 mm x 1 mm; Eluent A: 1 L Wasser + 0.5 mL 50%ige Ameisensäure, Eluent B: 1 L Acetonitril + 0.5 mL 50%ige Ameisensäure; Gradient: 0.0 min 90% A → 0.1 min 90% A → 1.5 min 10% A → 2.2 min 10% A; Ofen: 50°C; Fluss: 0.33 mL/min; UV-Detektion: 210 nm.

Methode 6 (LC-MS): Gerätetyp MS: Micromass ZQ; Gerätetyp HPLC: HP 1100 Series; UV DAD; Säule: Phenomenex Gemini 3µ 30 mm x 3.00 mm; Eluent A: 1 L Wasser + 0.5 mL 50%ige Ameisensäure, Eluent B: 1 L Acetonitril + 0.5 mL 50%ige Ameisensäure; Gradient: 0.0 min 90% A → 2.5 min 30% A → 3.0 min 5% A → 4.5 min 5% A; Fluss: 0.0 min 1 mL/min, 2.5 min/3.0 min/4.5 min 2 mL/min; Ofen: 50°C; UV-Detektion: 210 nm.

Methode 7 (GC-MS): Instrument: Micromass GCT, GC6890; Säule: Restek RTX-35, 15 m x 200 µm x 0.33 µm; konstanter Fluss mit Helium: 0.88 mL/min; Ofen: 70°C; Inlet: 250°C; Gradient: 70°C, 30°C/min → 310°C (3 min halten).

### Beispiel 1: Herstellung von 3-Brompyridon

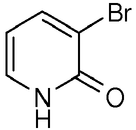

Zu einer Lösung von 5.0 kg (42.0 mol) Kaliumbromid und 1.60 kg (16.8 mol) 2-Hydroxypyridin wurde innerhalb von 2 Stunden bei 35-40°C 2.69 kg (16.8 mol) Brom dosiert. Es wurde 1 h bei 35°C gerührt und anschließend auf 22°C abgekühlt. Der pH-Wert wurde mit einer 23%igen Natronlauge auf pH 9.5 eingestellt und das Produkt über eine Nutsche isoliert. Es wurde zweimal mit je 2 L Wasser und zweimal mit je 1 L Wasser-Methanol 1:1 gewaschen. Das Produkt wurde im Vakuum bei 40°C bis zur Gewichtskonstanz getrocknet. Man erhielt 1.53 kg (52%) des gewünschten Produkts.
HPLC (Methode 1): Rₜ = 13.9 min mit 97Fl%.
¹H-NMR (500MHz, DMSO-d₆): δ [ppm]= 6.14 (t, 1H), 7.46 (dd, 1H), 7.92 (dd, 1H), 11.95 - 12.29 (m, 1H).
MS (TOF EI⁺): 173

### Beispiel 2: Herstellung von 3-Brom-1-(2-methyl-4-nitrophenyl)pyridin-2(1H)-on

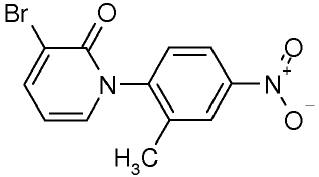

Eine Suspension von 125 g (718 mmol) 3-Brompyridon, 106 g (682 mmol) 2-Fluor-5-Nitrotoluol und 183 g (862 mmol) wasserfreies Trikaliumphoshat in 325 mL NMP wurde 4 h auf 120°C erhitzt. Bei 90°C wurde mit 1250 mL Wasser versetzt und es wurde unter Rühren auf 20°C abgekühlt. Es wurde 16 h nachgerührt und über eine Nutsche isoliert. Es wurde zweimal mit je 500 mL Wasser und zweimal mit je 500 mL Wasser/Methanol 1:1 gewaschen. Schließlich wurde im Vakuum bei 40°C bis zur Gewichtskonstanz getrocknet. Man erhielt 178 g (80%) des gewünschten Produkts. Man kann die Reinheit des Produkts durch eine Umkristallisation z.B. aus Dioxan oder Acetonitril weiter erhöhen auf ≥ 99 Fl%.
HPLC (Methode 1): Rₜ = 13.9 min mit 97 Fl%.
¹H-NMR (500MHz, DMSO-d₆): δ [ppm]= 2.17 (s, 3H), 6.37 (t, 1H), 7.65 (d, 1H), 7.69 (dd, 1H), 8.10 (dd, 1H), 8.21 (dd, 1H), 8.34 (d, 1H).
MS (DCI-NH₃): [M+H]⁺ 309

### Beispiel 3: Herstellung von [1-(2-Methyl-4-nitrophenyl)-2-oxo-1,2-dihydropyridin-3-yl]-essigsäure

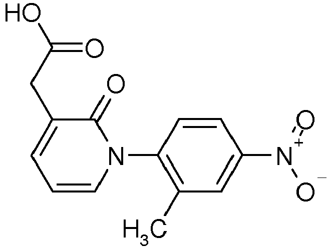

Zu einer Lösung von 110 g (1.14 mmol) Natrium*tert*butylat in 700 mL Dioxan wurde innerhalb 15 min 180 g Malonsäurediethylester zugetropft. Es wurde mit 200 mL Dioxan verdünnt und 1 h bei 20°C nachgerührt. Es wurden 7.5 g (24 mmol) Bis-(2-Pyridincarboxylato)Kupfer(II)-Komplex und 100 g (0.32 mol) 3-Brom-1-(2-methyl-4-nitrophenyl)pyridin-2(1H)-on zugegeben und auf 95°C erhitzt. Man destillierte innerhalb 3.5 h ca. 300 mL Dioxan ab, kühlte auf 20°C ab und versetzte mit 900 mL Wasser. Es wurde mit 108 mL konzentrierter Natronlauge versetzt und 1 h bei 45°C gerührt. Bei 20°C wurden 800 mL Dichlormethan zugegeben und es wurde über Kieselgur filtriert. Die organische Phase wurde verworfen, die Wasserphase wurde mit 1 L Dichlormethan versetzt und mit 37%iger Salzsäure auf pH 1 eingestellt. Es wurde 2 h bis zur Beendigung der Gasentwicklung gerührt. Die Wasserphase wurde mit 240 mL Dichlormethan extrahiert und die vereinigten organischen Phasen wurden auf ca. 250 mL aufkonzentriert. Es wurde mit 400 mL Dioxan versetzt und erneut auf ca. 250 mL aufkonzentriert. Dieser Arbeitsschritt wurde wiederholt. Es wurden 500 mL Methyl-tert-butylether zugesetzt und 1 h bei 0-5°C gerührt. Es wurde über eine Nutsche abgesaugt, zweimal mit je 100 mL Methyl-tert-butylether gewaschen und im Vakuum bis zur Gewichtskonstanz getrocknet. Man erhielt 74.3 g (80%) der gewünschten Carbonsäure.
HPLC (Methode 1): Rₜ = 10.3 min mit 99 Fl%.
¹H-NMR (400MHz, DMSO-d₆): δ [ppm]= 2.14 (s, 3H), 3.43 (s, 2H), 6.38 (t, 1H), 7.52 (t, 1H), 7.57 (d, 1H), 8.19 (dd, 1H), 8.31 (d, 1H).
MS (DCI-NH₃): [M+H]⁺ 289.

### Beispiel 4: Herstellung von 3-(2-Hydroxyethyl)-1-(2-methyl-4-nitrophenyl)pyridin-2(1H)-on

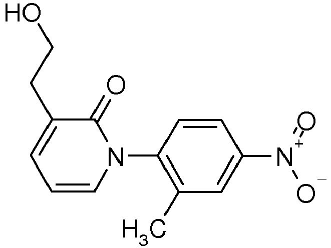

Zu einer Lösung von 50 g (174 mmol) [1-(2-Methyl-4-nitrophenyl)-2-oxo-1,2-dihydropyridin-3-yl]essigsäure in 100 mL 2-Methyl-THF wurden bei 20°C innerhalb von 20 min 56.8 g (348 mmol) Boran-Dimethylanilin Komplex zugetropft. Es wurde 16 h nachgerührt und mit 500 mL Dichlormethan verdünnt. Dieses Gemisch wurde innerhalb von 30 min auf eine Lösung von 50 g Natriumchlorid in 500 mL 1M Salzsäure dosiert. Es wurde 1 h nachgerührt und die organische Phase wurde erneut mit 500 mL einer Lösung von 50 g Natriumchlorid in 500 mL 1M Salzsäure gewaschen. Es wurde mit 500 mL einer gesättigten wässrigen Natriumhydrogencarbonat Lösung gewaschen, über Magnesiumsulfat getrocknet und eingedampft. Es wurden 40 g (84%) des gewünschten Alkohols erhalten.
HPLC (Methode 1): Rₜ = 11.2 min mit 100 Fl%.
¹H-NMR (400MHz, CDCl₃): δ [ppm]= 2.27 (s, 3H), 2.89 (t, 2H), 3.89 (t, 2H), 6.34 (t, 4H), 7.11 (dd, 1H), 7.41 (d, 1H), 8.20 (dd, 1H), 8.25 (d, 1H).
MS (DCI-NH₃): [M+H]⁺ 275.

### Beispiel 5: Herstellung von 3-Brom-1-(2-methoxy-4-nitrophenyl)pyridin-2(1H)-on

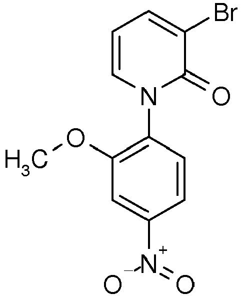

36.2 g 3-Brompyridon (87% Reinheit, 0.18 mol) und 29.4 g 4-Fluor-3-methoxynitrobenzol (0.17 mol) wurden in 180 mL N-Methylpyrrolidon vorgelegt, 46.1 g wasserfreies Kaliumphosphat (0.22 mol) hinzugegeben und das Reaktionsgemisch 6 h bei 120°C gerührt. Nach Abkühlung auf 90°C wurde Wasser hinzugefügt und auf Raumtemperatur abgekühlt. Der Niederschlag wurde abfiltriert, mit Wasser gewaschen und mit einem Methanol-Wasser Gemisch (1:1) verrührt. Der Feststoff wurde abfiltriert, gewaschen und an der Luft getrocknet. Man erhielt 38.7 g (66%) der gewünschten Zielverbindung.
HPLC (Methode 3): Rₜ = 3.50 min.
LC-MS (Methode 4): Rₜ = 0.84 min.
¹H-NMR (400MHz, DMSO-d₆): δ [ppm] = 3.91 (s, 3H), 6.30 (t, 1H), 7.64 (dd, 1H), 7.70 (d, 1H), 7.96 (dd, 1H), 8.00 (m, 1H), 8.04 (dd, 1H).
MS (ES+): [M+H]⁺ 327 bzw. 325.

### Beispiel 6: Herstellung von Diethyl-[1-(2-methoxy-4-nitrophenyl)-2-oxo-1,2-dihydropyridin-3-yl]malonat

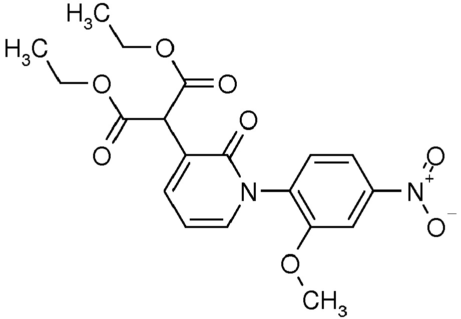

39.3 g Natrium-tert.-butylat (0.41 mol) wurden in 300 mL Dioxan vorgelegt und 65.5 g Malonsäurediethylester (0.41 mol) binnen 15 min so zugetropft, dass die Temperatur 30°C nicht überschritt. Nach 1 h wurden 2.70 g (8.8 mmol) Bis-(2-Pyridincarboxylato)Kupfer(II)-Komplex sowie 38.0 g (0.12 mol) 3-Brom-1-(2-methoxy-4-nitrophenyl)pyridin-2(1H)-on hinzugefügt und die Reaktionsmischung 8 h zum Rückfluss erhitzt. Nach Abkühlung auf RT wurde über Nacht weitergerührt. Die Reaktionsmischung wurde vom Feststoff abgesaugt, der Rückstand mit Dioxan nachgewaschen und die vereinten organischen Fraktionen bis auf ca. 300 g am Rotationsverdampfer eingeengt. Die Rohlösung wurde ohne weitere Aufreinigung in die nächste Stufe eingesetzt.
LC-MS (Methode 4): Rₜ = 1.00 min.
MS (ES+): [M+H]⁺ 405

### Beispiel 7: Herstellung von [1-(2-Methoxy-4-nitrophenyl)-2-oxo-1,2-dihydropyridin-3-yl]essigsäure

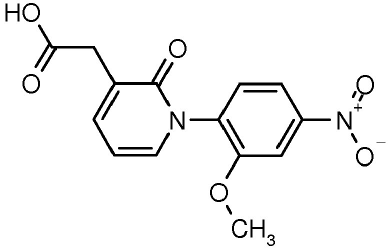

Die Rohproduktlösung von Diethyl-[1-(2-methoxy-4-nitrophenyl)-2-oxo-1,2-dihydropyridin-3-yl]malonat (maximal 0.12 mol) wurde mit 390 mL Wasser verdünnt und langsam 39 mL konzentrierte Natronlauge hinzugegeben. Das Reaktionsgemisch wurde 90 min bei 45°C gerührt, auf RT abgekühlt mit Wasser verdünnt und dreimal mit Dichlormethan extrahiert. Die organische Phase wurde verworfen, die wässrige Phase wurde mit 300 mL Dichlormethan versetzt und bei 15°C mit konzentrierter Salzsäure auf pH 1 gestellt. Bereits im schwach sauren Milieu tritt Kohlendioxid-Entwicklung ein. Nach Erwärmung auf RT wurde 1 h gerührt, dann die Phasen getrennt und die organische Phase über Natriumsulfat getrocknet, filtriert und am Rotationsverdampfer zur Trockne eingeengt. Der Rückstand wurde mit Acetonitril verrührt, abgesaugt, nachgewaschen und im Vakuum getrocknet. Man erhielt 17.4 g (49%) der gewünschten Verbindung. Die Mutterlauge wurde zur Trockne eingeengt, mit Acetontril verrührt und analog aufgearbeitet. Dabei erhielt man weitere 3.6 g (10%) der gewünschten Verbindung.
HPLC (Methode 2): Rₜ = 3.17 min.
LC-MS (Methode 4): Rₜ = 0.69 min.
LC-MS (Methode 5): Rₜ = 0.75 min.
¹H-NMR (400MHz, DMSO-d₆): δ [ppm] = 3.40 (s, 2H), 3.90 (s, 3H), 6.31 (t, 1H), 7.45-7.50 (m, 2H), 7.61 (d, 1H), 7.94 (dd, 1H), 7.98 (m, 1H), 12.21 (br. s, 1H).
MS (ES+): [M+H]⁺ 305.

### Beispiel 8: Herstellung von 3-(2-Hydroxyethyl)-1-(2-methoxy-4-nitrophenyl)pyridin-2(1H)-on

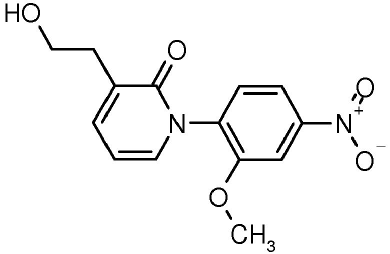

21.0 g (69.0 mmol) [1-(2-Methoxy-4-nitrophenyl)-2-oxo-1,2-dihydropyridin-3-yl]essigsäure wurden in 210 mL THF vorgelegt und 104 mL 1M Boran-Lösung in THF bei RT hinzugetropft. Die Temperatur stieg dabei auf 27°C. Das Reaktionsgemisch wurde über Nacht bei RT gerührt. Nach der Verdünnung mit 400 mL Dichlormethan wurden unter Kühlung 400 mL 1N Natronlauge hinzugegeben und 30 min gerührt. Die Phasen wurden getrennt, die organische Phase mit Wasser gewaschen, über Natriumsulfat getrocknet, filtriert und am Rotationsverdampfer zur Trockne eingeengt. Es wurden 17.0 g (85%) Rohprodukt erhalten, die ohne Aufreinigung weiter umgesetzt wurden.
LC-MS (Methode 5): Rₜ = 0.76 min.
MS (ES+): [M+H]⁺ 291.

### Beispiel 9: Herstellung von 2-(Brommethyl)-1-fluor-4-nitrobenzol

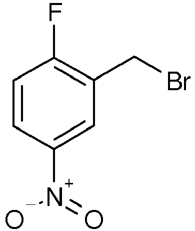

### Methode Benzylalkohol / Phosphortribromid

70.0 g (0.41 mol) 2-Fluor-5-nitro-benzylalkohol wurden in 470 mL Chloroform vorgelegt und 55.4 g (0.21 mol) Phosphortribromid hinzugegeben. Dabei stieg die Temperatur auf 37°C. Das Reaktionsgemisch wurde 30 min bei 60°C gerührt. Nach Abkühlung auf RT wurde durch Zugabe von gesättigter wässriger Natriumhydrogencarbonat-Lösung neutralisiert (pH = 7). Die organische Phase wurde abgetrennt und die wässrige mit 50 mL Dichlormethan extrahiert. Die vereinten organischen Phasen wurden über Natriumsulfat getrocknet, filtriert und am Rotationsverdampfer zur Trockne eingeengt. Der erhaltene Feststoff wurde im Vakuum getrocknet. Man erhielt 93.7 g (98%) der gewünschten Verbindung, die ohne Aufreinigung weiter umgesetzt wurde.

### Methode N-Bromsuccinimid / Toluol

905 g (5.83 mol) 2-Fluor-5-nitrotoluol und 1038 g (5.83 mol) N-Bromsuccinimid wurden in 5.83 L Chloroform vorgelegt, 47.8 g (0.29 mol) 2,2'-Azobis[isobutyronitril] hinzugegeben und das Reaktionsgemisch 18 h zum Rückfluss erhitzt. Nach dem Abkühlen auf RT wurde vom Feststoff abgesaugt und das Filtrat in drei Portionen aufgeteilt. Diese wurden jeweils auf 1.5 kg Kieselgel (0.06-0.2) aufgezogen und an jeweils an 10 kg Kieselgel mit 160 L Essigsäureethylester : Petrolether (2.5 : 97.5) chromatographisch gereinigt. Die Produktfraktionen wurden vereinigt, am Rotationsverdampfer zur Trockne eingeengt und der Rückstand mit Petrolether 10 min ausgerührt. Der Feststoff wurde abgesaugt, mit Petrolether nachgewaschen und im Vakuum getrocknet. Es wurden 385 g (28%) erhalten.
HPLC (Methode 3): Rₜ = 3.95 min.
¹H-NMR (400MHz, DMSO-d₆): δ [ppm] = 4.82 (s, 2H), 7.57 (t, 1H), 8.30 (ddd, 1H), 8.55 (dd, 1H).
MS (EI+): [M-Br]⁺ 154.

### Beispiel 10: Herstellung von 1-Fluor-2-(methoxymethyl)-4-nitrobenzol

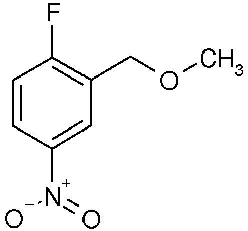

Unter Argon wurden 250 g (1.07 mol) 2-(Brommethyl)-1-fluor-4-nitrobenzol in 2.51 L Methanol vorgelegt, 371 g (1.60 mol) Silber-(I)-oxid hinzugefügt und das Reaktionsgemisch 2.5 h bei 52-56°C gerührt. Nach dem Abkühlen auf RT wurde über Kieselgur abgesaugt, mit Methanol nachgewaschen und das Lösemittel am Rotationsverdampfer entfernt. Das Rohprodukt wurde am Kurzwegverdampfer bei 104°C Manteltemperatur und 0.56 mbar destillativ gereinigt. Man erhielt 184.5 g (93%) des gewünschten Produktes.
HPLC (Methode 3): Rₜ = 3.99 min.
GC-MS (EI) (Methode 7): Rₜ = 4.52 min.
¹H-NMR (400MHz, DMSO-d₆): δ [ppm] = 3.37 (s, 3H), 4.57 (s, 2H), 7.52 (t, 1H), 8.25-8.33 (m, 2H).
MS (EI+): [M]⁺ 185 (5%), 184 (45%) [M-OMe]⁺ 154 (90%).

### Beispiel 11: Herstellung von 3-Brom-1-[2-(methoxymethyl)-4-nitrophenyl]pyridin-2(1H)-on

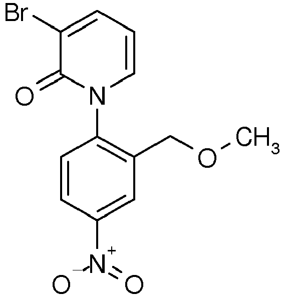

Unter Argon wurden 44.2 g (85% Reinheit, 216 mmol) 3-Brompyridon in 450 mL N-Methylpyrrolidon vorgelegt und 29.1 g Kalium-tert.-butanolat (259 mmol) portionsweise so zugegeben, dass die Temperatur unter 30°C blieb. Es wurde 1 h gerührt und dann 40 g (216 mmol) 1-Fluor-2-(methoxymethyl)-4-nitrobenzol hinzugegeben. Das Reaktionsgemisch wurde 4 h bei 80°C gerührt, auf RT abgekühlt und in eine Eis/Wasser Mischung eingerührt. Der pH-Wert wurde mit halbkonzentrierter Salzsäure auf pH = 4 gestellt, mit Essigsäureethylester extrahiert, die organische Phase über Natriumsulfat getrocknet, filtriert und am Rotationsverdampfer zur Trockne eingeengt. Der Rückstand wurde in 2 L Dichlormethan gelöst, auf die fünffache Menge Kieselgel aufgezogen und an Kieselgel mit Essigsäureethylester : Dichlormethan 3 : 97 und 5 : 95 chromatographisch gereinigt. Die Produktfraktionen wurden am Rotationsverdampfer zur Trockne eingeengt und der Rückstand im Vakuum getrocknet. Man erhielt 55 g (73%) des gewünschten Produktes.
HPLC (Methode 3): Rₜ = 3.50 min.
LC-MS (Methode 6): Rₜ = 1.95 min.
¹H-NMR (400MHz, DMSO-d₆): δ [ppm] = 3.26 (s, 3H), 4.27 (d, 1H), 4.34 (d, 1H), 6.35 (t, 1H), 7.69 (dd, 1H), 7.71 (d, 1H), 8.09 (dd, 1H), 8.32 (dd, 1H), 8.37 (d, 1H).
MS (ES+): [M+H]⁺ 339 bzw. 341.

### Beispiel 12: Herstellung von Diethyl-{1-[2-(methoxymethyl)-4-nitrophenyl]-2-oxo-1,2-dihydropyridin-3-yl}malonat

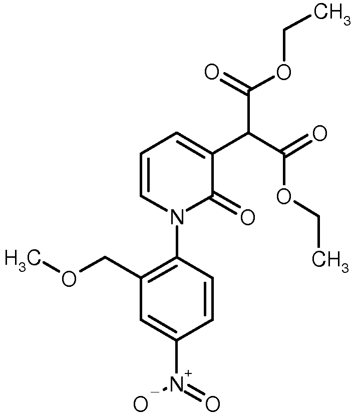

Unter Argon wurde eine Suspension von 49.3 g (513 mmol) Kalium-tert.-butanolat bei RT in 360 mL Dioxan vorgelegt und 82.2 g (513 mmol) Malonsäurediethylester so zugegeben, dass die Temperatur unter 30°C blieb. Das Reaktionsgemisch wurde 1 h bei RT gerührt, 3.4 g (11.0 mmol) Bis-(2-Pyridincarboxylato)Kupfer(II)-Komplex und 49.7 g (147 mmol) 3-Brom-1-[2-(methoxy-methyl)-4-nitrophenyl]pyridin-2(1H)-on hinzugefügt und 4.5 h bei 95°C gerührt. Es wurden weitere 2.0 g (6.5 mmol) Bis-(2-Pyridincarboxylato)Kupfer(II)-Komplex hinzugegeben und das Reaktionsgemisch über Nacht bei 95°C gerührt. Nach dem Abkühlen auf RT wurde mit 0.9 L Essigsäureethylester verdünnt, 1.8 L 3N Salzsäure zugegeben und 15 min bei RT gerührt. Die wässrige Phase wurde mit 0.9 L Essigsäureethylester extrahiert und die vereinten organischen Phasen mit gesättigter wässriger Natriumchloridlösung gewaschen. Nach der Trocknung über Natriumsulfat, Filtration und Entfernen des Lösemittels am Rotationsverdampfer wurde der Rückstand im Vakuum getrocknet. Man erhielt 115 g Rohprodukt, welches ohne Aufreinigung weiter umgesetzt wurde.
HPLC (Methode 3): Rₜ = 3.96 min.
LC-MS (Methode 4): Rₜ = 1.01 min
¹H-NMR (400MHz, DMSO-d₆): δ [ppm]= 1.18 (t, 3H), 1.20 (t, 3H), 3.24 (s, 3H), 4.11 - 4.25 (m, 5H), 4.31 (d, 1H), 4.84 (s, 1H), 6.46 (t, 1H), 7.56 (dd, 1H), 7.65 (dd, 1H), 7.67 (d, 1H), 8.30 - 8.34 (m, 1H), 8.35 (d, 1H).
MS (ES+): [M+H]⁺ 419.

### Beispiel 13: Herstellung von {1-[2-(Methoxymethyl)-4-nitrophenyl]-2-oxo-1,2-dihydropyridin-3-yl}essigsäure

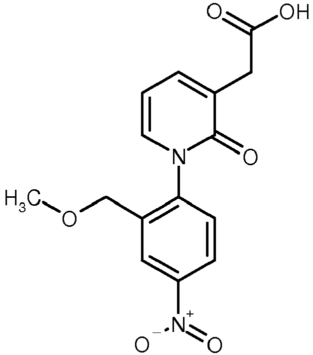

115 g (maximal 147 mmol) des Rohproduktes von Diethyl-{1-[2-(methoxymethyl)-4-nitrophenyl]-2-oxo-1,2-dihydropyridin-3-yl}malonat wurden in 470 mL Dioxan bei RT vorgelegt, 940 mL 2N Natronlauge hinzugegeben und das Reaktionsgemisch 1.5 h bei RT gerührt. Durch Zugabe von 313 mL konzentrierter Salzsäure wurde der pH = 1 eingestellt und das Gemisch anschließend 2 h bei 50°C gerührt. Nach dem Abkühlen auf RT wurde dreimal mit je 1.17 L Essigsäureethylester extrahiert. Die vereinten organischen Phasen wurden dreimal mit je 780 mL gesättigter wässriger Natriumchloridlösung gewaschen, über Natriumsulfat getrocknet, filtriert und am Rotationsverdampfer zur Trockne eingeengt. Der Rückstand wurde in wenig Dichlormethan gelöst und an 1.96 kg Kieselgel (0.04-0.06 mm) mit Methanol : Dichlormethan 5 : 95 und 1 : 9 chromatographisch gereinigt. Die Produktfraktionen wurden am Rotationsverdampfer zur Trockne eingeengt. Es wurden 34.9 g (75% über 2 Stufen) des gewünschten Produktes als Öl isoliert.
HPLC (Methode 3): Rₜ = 3.16 min.
¹H-NMR (400MHz, DMSO-d₆): δ [ppm] = 3.25 (s, 3H), 3.43 (s, 2H), 4.21 (d, 1H), 4.31 (d, 1H), 6.37 (t, 1H), 7.53 (m, 2H), 7.64 (d, 1H), 8.30 (dd, 1H), 8.34 (m, 1H), 12.27 (br. s, 1H).
MS (ES+): [M+H]⁺ 319.

### Beispiel 14: Herstellung von 3-(2-Hydroxyethyl)-1-[2-(methoxymethyl)-4-nitrophenyl]pyri-din-2(1H)-on

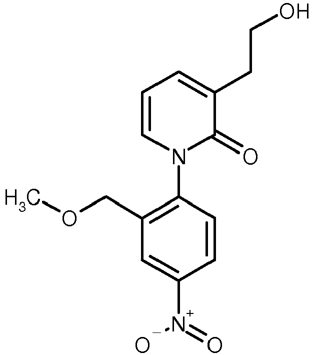

Unter Argon wurden 37.7 g (109 mmol) {1-[2-(Methoxymethyl)-4-nitrophenyl]-2-oxo-1,2-dihydropyridin-3-yl}essigsäure in 310 mL Tetrahydrofuran bei RT vorgelegt und 53.3 g (327 mmol) Borandiethylanilin-Komplex zunächst vorsichtig dann nach dem Abklingen des starken Schäumens zügig hinzugegeben. Das Reaktionsgemisch wurde über Nacht bei RT gerührt, mit 500 mL Essigsäureethylester verdünnt und mit 1N Salzsäure (1.55 L) zunächst vorsichtig (starke Schaumentwicklung) dann zügig versetzt. Das Reaktionsgemisch wurde 30 min nachgerührt, die organische Phase abgetrennt und die wässrige Phase noch einmal mit 500 mL Essigsäureethylester extrahiert. Die vereinten organischen Phasen wurden zweimal mit je 500 mL 1N Salzsäure gewaschen, einmal mit 200 mL 1N Natronlauge gewaschen, über Natriumsulfat getrocknet, filtriert und am Rotationsverdampfer zur Trockne eingeengt. Der Rückstand wurde zunächst an 500 g Kieselgel (0.04-0.06 mm) mit Methanol : Dichlormethan 5 : 95 dann 1 : 9 und nochmals an 500 g Kieselgel (0.04-0.06 mm) mit Aceton : Dichlormethan 3 : 7 chromatographisch gereinigt. Dabei wurden 14.2 g (43%) des gewünschten Produktes erhalten.
HPLC (Methode 3): Rₜ = 3.19 min.
¹H-NMR (400MHz, DMSO-d₆): δ [ppm] = 2.61 (t, 2H), 3.26 (s, 3H), 3.59 (q, 2H), 4.22 (d, 1H), 4.32 (d, 1H), 4.61 (t, 1H), 6.34 (t, 1H), 7.46 (d, 2H), 7.62 (d, 1H), 8.30 (dd, 1H), 8.35 (m, 1H).
MS (DCI, NH₃): [M+H]⁺ 305.

### Beispiel 15: Herstellung von 3-Brom-1-(2,6-dimethyl-4-nitrophenyl)pyridin-2(1H)-on

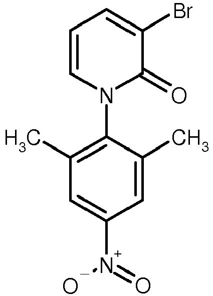

223 g (38%ig, 0.5 mol) 2-Fluor-1,3-dimethyl-5-nitrobenzol und 138 g (1.0 mol) Kaliumcarbonat wurden in 1.95 L DMSO vorgelegt und auf 120°C erwärmt. Bei dieser Temperatur wurde eine Lösung von 100 g (87% Reinheit, 0.5 mol) 3-Brompyridon in DMSO hinzugetropft. Das Reaktionsgemisch wurde 3.5 h bei 120°C gerührt, auf RT abgekühlt, in Wasser eingerührt, mit Salzsäure leicht sauer gestellt und mit Essigsäureethylester extrahiert. Die wässrige Phase wurde nochmals mit Essigsäureethylester extrahiert, die vereinten organischen Phasen mit Wasser gewaschen, über Natriumsulfat getrocknet, filtriert und am Rotationsverdampfer zur Trockne eingeengt. Das Rohprodukt wurde an Kieselgel mit Dichlormethan dann Essigsäureethylester / Dichlormethan = 1 : 20 chromatographisch gereinigt. Das isolierte Produkt wurde in Diethylether / Petrolether = 1 : 1.5 verrührt, abgesaugt und mit Petrolether gewaschen. Man erhielt 75.2 g (47%) des gewünschten Produktes.
HPLC (Methode 3): Rₜ = 4.17 min.
¹H-NMR (400MHz, DMSO-d₆): δ [ppm] = 2.10 (s, 6H), 6.41 (t, 1H), 7.63 (dd, 1H), 8.14 (dd, 1H), 8.18 (s, 2H).
MS (ES+): [M+H]⁺ 323 bzw. 325.

### Beispiel 16: Herstellung von Diethyl-[1-(2,6-dimethyl-4-nitrophenyl)-2-oxo-1,2-dihydropyridin-3-yl]malonat

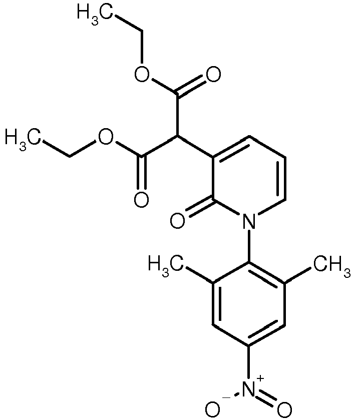

Unter Argon wurden 42.5 g (379 mmol) Kalium-tert.-Butylat in 530 mL Dioxan suspendiert und auf 55-60°C erwärmt. Bei dieser Temperatur wurde eine Lösung von 60.7 g (379 mmol) Malonsäurediethylester in 100 mL Dioxan über 20 min addiert. Nach beendeter Zugabe wurde das Reaktionsgemisch 1 h bei 60°C gerührt. Danach wurden 6.72 g (21.7 mmol) Bis-(2-Pyridincarboxylato)Kupfer(II)-Komplex hinzugegeben sowie 35.0 g (108 mmol) 3-Brom-1-(2,6-dimethyl-4-nitrophenyl)pyridin-2(1H)-on. Das Reaktionsgemisch wurde 8 h zum Rückfluss erwärmt und danach über Nacht bei RT gerührt. Es wurde vom Feststoff abgesaugt, mit Dioxan nachgewaschen und das Filtrat bis auf 250 g am Rotationsverdampfer eingeengt. Die Rohlösung wurde ohne Aufreinigung weiter umgesetzt.
LC-MS (Methode 5): Rₜ = 1.18 min.
MS (ES+): [M+H]⁺ 403.

### Beispiel 17: Herstellung von [1-(2,6-Dimethyl-4-nitrophenyl)-2-oxo-1,2-dihydropyridin-3-yl]essigsäure

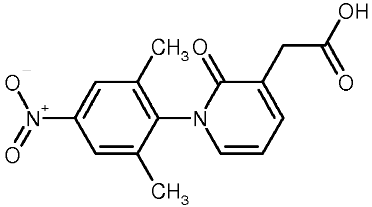

Die Rohproduktlösung von Diethyl-[1-(2,6-dimethyl-4-nitrophenyl)-2-oxo-1,2-dihydropyridin-3-yl]malonat in Dioxan (maximal 108 mmol) wurde in 350 mL Wasser vorgelegt und 35 mL konzentrierte Natronlauge langsam hinzugegeben. Das Reaktionsgemisch wurde 90 min bei 45°C gerührt. Nach dem Abkühlen auf RT wurden 100 mL Wasser addiert, dreimal mit Dichlormethan gewaschen und die wässrige Phase (pH = 14) mit 270 mL Dichlormethan versetzt. Bei 10-15°C wurden 59.5 mL konzentrierte Salzsäure langsam hinzugegeben bis zum pH = 1. Bereits bei schwach saurem pH setzte die Kohlendioxid-Entwicklung ein. Das Reaktionsgemisch wurde auf RT erwärmt und 1 h, bis zum Ende der Gasentwicklung, gerührt. Nach der Phasentrennung wurde die organische Phase über Natriumsulfat getrocknet, filtriert und am Rotationsverdampfer zur Trockne eingeengt. Der Rückstand wurde mit 200 mL Methyl-tert.-butylether 30 min verrührt. Der kristalline Feststoff wurde abgesaugt, mit Methyl-tert.-butylether gewaschen und im Vakuum getrocknet. Es wurden 14.8 g (47% über 2 Stufen) des gewünschten Produktes erhalten. Die Mutterlauge wurde weitgehend eingeengt, die Nachfällung abgesaugt, nachgewaschen und im Vakuum getrocknet. Es wurden weitere 5.3 g (17% über 2 Stufen) des gewünschten Produktes erhalten.
HPLC (Methode 3): Rₜ = 3.39 min.
¹H-NMR (400MHz, DMSO-d₆): δ [ppm] = 2.09 (s, 6H), 3.44 (s, 2H), 6.41 (t, 1H), 7.42 (dd, 1H), 7.54 (dd, 1H), 8.15 (s, 2H), 12.18 (br. s, 1H).
MS (ES+): [M+H]⁺ 303.

### Beispiel 18: Herstellung von 1-(2,6-Dimethyl-4-nitrophenyl)-3-(2-hydroxyethyl)pyridin-2(1H)-on

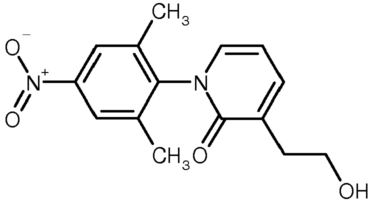

26.5 g (87.7 mmol) [1-(2,6-Dimethyl-4-nitrophenyl)-2-oxo-1,2-dihydropyridin-3-yl]essigsäure wurden in 265 mL THF bei RT vorgelegt. Es wurden 132 mL 1M Boran-Lösung in THF (132 mmol) hinzugetropft. Die Temperatur stieg dabei auf 27°C. Das Reaktionsgemisch wurde 1 h bei RT gerührt und dann die gleiche Menge Boran-Lösung hinzugegeben. Das Reaktionsgemisch wurde über Nacht bei RT gerührt. Es wurde mit 500 mL Dichlormethan verdünnt und unter Kühlung 500 mL 1N Natronlauge hinzugegeben. Das Reaktionsgemisch wurde 30 min gerührt, die Phasen getrennt und die organische Phase mit Wasser gewaschen, über Natriumsulfat getrocknet, filtriert und am Rotationsverdampfer zur Trockne eingeengt. Man erhielt 12.6 g (48%) des gewünschten Produktes als kristallinen Feststoff.
HPLC (Methode 3): Rₜ = 3.88 min.
¹H-NMR (400MHz, DMSO-d₆): δ [ppm] = 2.09 (s, 6H), 2.63 (t, 2H), 3.58 (q, 2H), 4.61 (t, 1H), 6.37 (t, 1H), 7.35 (dd, 1H), 7.47 (dd, 1H), 8.15 (s, 2H).
MS (ES+): [M+H]⁺ 291.

## Patentansprüche

1. Verfahren zur Herstellung einer Verbindung der Formel 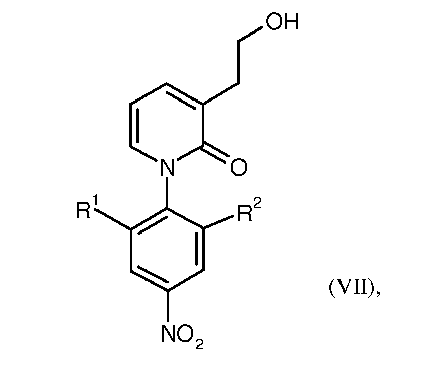 in welcher
R¹ für Wasserstoff oder C₁-C₃-Alkyl steht
und
R² für C₁-C₃-Alkyl steht,
oder
R¹ für Wasserstoff steht
und
R² für C₁-C₃-Alkoxy oder C₁-C₃-Alkoxymethyl steht,
**dadurch gekennzeichnet, dass** die Carboxyl-Gruppe in einer Verbindung der Formel 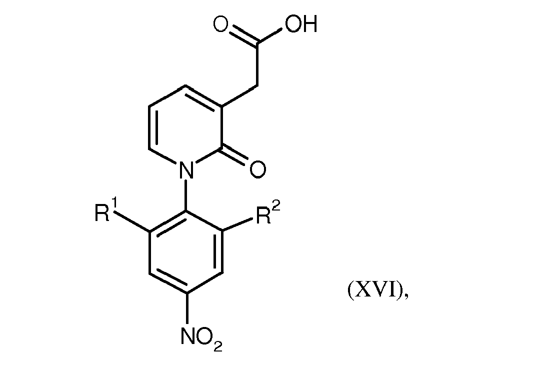 in welcher
R¹ für Wasserstoff oder C₁-C₃-Alkyl steht
und
R² für C₁-C₃-Alkyl steht,
oder
R¹ für Wasserstoff steht
und
R² für C₁-C₃-Alkoxy oder C₁-C₃-Alkoxymethyl steht,
in Gegenwart eines Boran-Komplexes reduziert wird.

2. Verfahren nach Anspruch 1, **dadurch gekennzeichnet, dass** zur Herstellung einer Verbindung der Formel (XVI) eine Verbindung der Formel 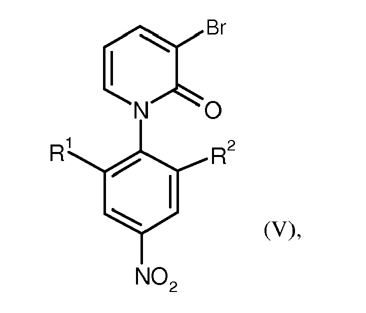 in welcher
R¹ für Wasserstoff oder C₁-C₃-Alkyl steht
und
R² für C₁-C₃-Alkyl steht,
oder
R¹ für Wasserstoff steht
und
R² für C₁-C₃-Alkoxy oder C₁-C₃-Alkoxymethyl steht,
in einem Eintopfverfahren zunächst in Gegenwart eines Kupferkatalysators mit einem Malonsäureester und einer Base und anschließend mit einer Base und Wasser umgesetzt wird.

3. Verfahren zur Herstellung einer Verbindung der Formel 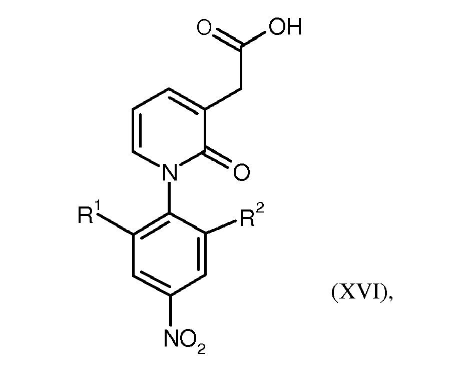 in welcher
R¹ für Wasserstoff oder C₁-C₃-Alkyl steht
und
R² für C₁-C₃-Alkyl steht,
oder
R¹ für Wasserstoff steht
und
R² für C₁-C₃-Alkoxy oder C₁-C₃-Alkoxymethyl steht,
**dadurch gekennzeichnet, dass** eine Verbindung der Formel 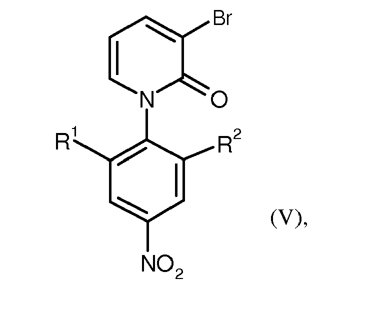 in welcher
R¹ für Wasserstoff oder C₁-C₃-Alkyl steht
und
R² für C₁-C₃-Alkyl steht,
oder
R¹ für Wasserstoff steht
und
R² für C₁-C₃-Alkoxy oder C₁-C₃-Alkoxymethyl steht,
in einem Eintopfverfahren zunächst in Gegenwart eines Kupferkatalysators mit einem Malonsäureester und einer Base und anschließend mit einer Base und Wasser umgesetzt wird.

4. Verfahren nach einem der Ansprüche 1, 2 oder 3, **dadurch gekennzeichnet, dass** in einer Verbindung der Formel (VII) R¹ für Methyl und R² für Methyl steht, in einer Verbindung der Formel (XVI) R¹ für Methyl und R² für Methyl steht und in einer Verbindung der Formel (V) R¹ für Methyl und R² für Methyl steht.

5. Verfahren nach einem der Ansprüche 1, 2 oder 4, **dadurch gekennzeichnet, dass** der Boran-Komplex Boran-Dimethylsulfid, Boran-Tetrahydrofuran, Boran-Diethylanilin oder Catecholboran ist.

6. Verfahren nach einem der Ansprüche 1, 2, 4 oder 5, **dadurch gekennzeichnet, dass** der Boran-Komplex im Überschuss eingesetzt wird.

7. Verfahren nach einem der Ansprüche 2, 3 oder 4, **dadurch gekennzeichnet, dass** der Kupferkatalysator ein vorab hergestellter Bis-(2-Pyridincarboxylato)Kupfer(II)-Komplex oder dessen verschiedene Hydratformen ist, oder aus Kupferiodid, Kupferchlorid, Kupfer(II)bromid, Kupfer(II)triflat oder Kupfer(II)sulfat, mit 2-Pyridincarbonsäure in situ hergestellt wurde.

8. Verfahren nach Anspruch 7, **dadurch gekennzeichnet, dass** der Kupferkatalysator ein vorab hergestellter Bis-(2-Pyridincarboxylato)Kupfer(II)-Komplex oder dessen verschiedene Hydratformen ist.

9. Verfahren nach einem der Ansprüche 2, 3 oder 4, **dadurch gekennzeichnet, dass** der Malonsäureester Diethylmalonsäureester, Dibenzylmalonsäureester, Di-tert-butylmalonsäureester oder Kalium-ethylmalonsäureester ist.

10. Verfahren nach Anspruch 9, **dadurch gekennzeichnet, dass** der Malonsäureester Diethylmalonsäureester ist.

11. Verfahren nach einem der Ansprüche 2, 3 oder 4, **dadurch gekennzeichnet, dass** die Base Natrium- oder Kalium-tert.-butylat, Kalium-, Natrium- oder Cäsiumcarbonat, Cäsiumfluorid oder Natriumhydrid ist.

12. Verfahren nach Anspruch 11, **dadurch gekennzeichnet, dass** die Base Natrium- oder Kalium-tert.-butylat ist.

13. Verbindung der Formel 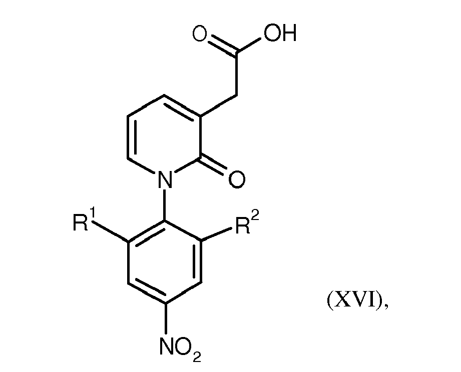 in welcher
R¹ für Wasserstoff oder C₁-C₃-Alkyl steht
und
R² für C₁-C₃-Alkyl steht,
oder
R¹ für Wasserstoff steht
und
R² für C₁-C₃-Alkoxy oder C₁-C₃-Alkoxymethyl steht,
oder eines ihrer Salze, ihrer Solvate oder der Solvate ihrer Salze.

14. Verbindung nach Anspruch 13, **dadurch gekennzeichnet, dass** R¹ für Methyl steht und R² für Methyl steht.

## Claims

1. Process for the preparation of a compound of the formula, 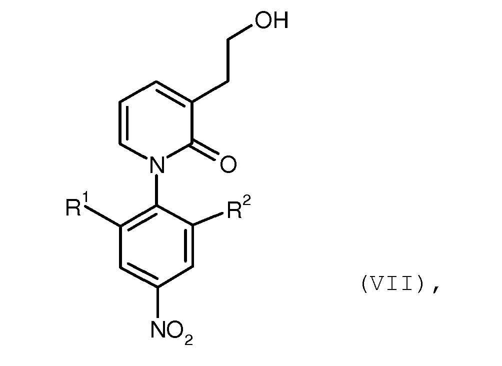 in which
R¹ is hydrogen or C₁-C₃-alkyl
and
R² is C₁-C₃-alkyl,
or
R¹ is hydrogen
and
R² is C₁-C₃-alkoxy or C₁-C₃-alkoxymethyl,
**characterized in that** the carboxyl group in a compound of the formula 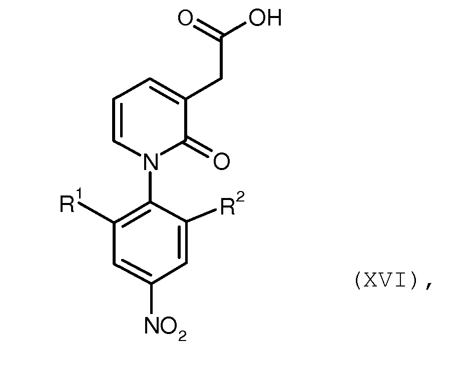 in which
R¹ is hydrogen or C₁-C₃-alkyl
and
R² is C₁-C₃-alkyl,
or
R¹ is hydrogen
and
R² is C₁-C₃-alkoxy or C₁-C₃-alkoxymethyl,
is reduced in the presence of a borane complex.

2. Process according to Claim 1, **characterized in that**, for the preparation of a compound of the Formula (XVI), a compound of the formula 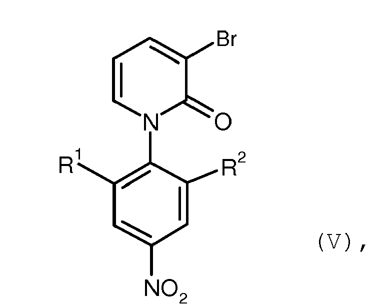 in which
R¹ is hydrogen or C₁-C₃-alkyl
and
R² is C₁-C₃-alkyl,
or
R¹ is hydrogen
and
R² is C₁-C₃-alkoxy or C₁-C₃-alkoxymethyl,
is reacted in a one-pot process firstly in the presence of a copper catalyst with a malonic acid ester and a base and then with a base and water.

3. Process for the preparation of a compound of the formula 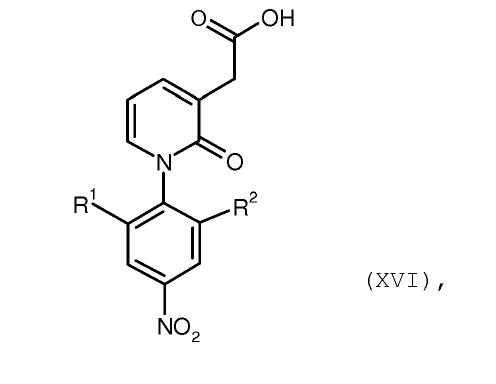 in which
R¹ is hydrogen or C₁-C₃-alkyl
and
R² is C₁-C₃-alkyl,
or
R¹ is hydrogen
and
R² is C₁-C₃-alkoxy or C₁-C₃-alkoxymethyl,
**characterized in that** a compound of the formula 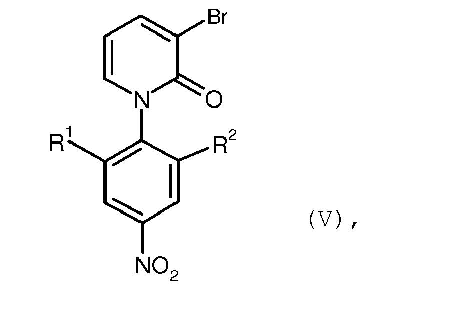 in which
R¹ is hydrogen or C₁-C₃-alkyl
and
R² is C₁-C₃-alkyl,
or
R¹ is hydrogen
and
R² is C₁-C₃-alkoxy or C₁-C₃-alkoxymethyl,
is reacted in a one-pot process firstly in the presence of a copper catalyst with a malonic acid ester and a base and then with a base and water.

4. Process according to one of Claims 1, 2 or 3, **characterized in that**, in a compound of the Formula (VII), R¹ is methyl and R² is methyl, in a compound of the Formula (XVI) R¹ is methyl and R² is methyl and in a compound of the Formula (V) R¹ is methyl and R² is methyl.

5. Process according to one of Claims 1, 2 or 4, **characterized in that** the borane complex is borane-dimethyl sulphide, borane-tetrahydrofuran, borane diethylaniline or catecholborane.

6. Process according to one of Claims 1, 2, 4 or 5, **characterized in that** the borane complex is used in excess.

7. Process according to one of Claims 2, 3 or 4, **characterized in that** the copper catalyst is a bis(2-pyridinecarboxylato)copper(II) complex prepared beforehand or its various hydrate forms, or has been prepared in-situ from copper iodide, copper chloride, copper(II) bromide, copper(II) triflate or copper(II) sulphate with 2-pyridinecarboxylic acid.

8. Process according to Claim 7, **characterized in that** the copper catalyst is a bis(2-pyridinecarboxylato)copper(II) complex produced beforehand or its various hydrate forms.

9. Process according to one of Claims 2, 3 or 4, **characterized in that** the malonic acid ester is diethylmalonic acid ester, dibenzylmalonic acid ester, di-tert-butylmalonic acid ester or potassium ethylmalonic acid ester.

10. Process according to Claim 9, **characterized in that** the malonic acid ester is diethylmalonic acid ester.

11. Process according to one of Claims 2, 3 or 4, **characterized in that** the base is sodium or potassium tert-butylate, potassium, sodium or caesium carbonate, caesium fluoride or sodium hydride.

12. Process according to Claim 11, **characterized in that** the base is sodium or potassium tert-butylate.

13. Compound of the formula 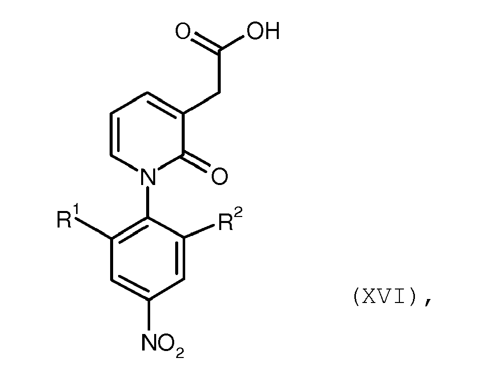 in which
R¹ is hydrogen or C₁-C₃-alkyl
and
R² is C₁-C₃-alkyl,
or
R¹ is hydrogen
and
R² is C₁-C₃-alkoxy or C₁-C₃-alkoxymethyl,
or one of its salts, its solvates or the solvates of its salts.

14. Compound according to Claim 13, **characterized in that** R¹ is methyl and R² is methyl.

## Revendications

1. Procédé pour la préparation d'un composé de formule 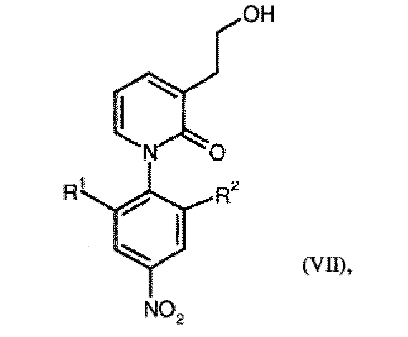 dans laquelle
R¹ représente un atome d'hydrogène ou un groupe alkyle en C₁-C₃
et
R² représente un groupe alkyle en C₁-C₃,
ou
R¹ représente un atome d'hydrogène
et
R² représente un groupe alcoxy en C₁-C₃ ou alcoxy(C₁-C₃)méthyle,
**caractérisé en ce qu'**on réduit le groupe carboxy dans un composé de formule 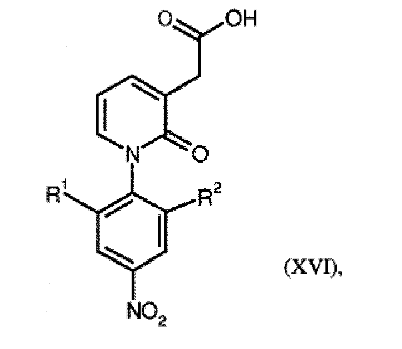 dans laquelle
R¹ représente un atome d'hydrogène ou un groupe alkyle en C₁-C₃
et
R² représente un groupe alkyle en C₁-C₃,
ou
R¹ représente un atome d'hydrogène
et
R² représente un groupe alcoxy en C₁-C₃ ou alcoxy(C₁-C₃)méthyle,
en présence d'un complexe de borane.

2. Procédé selon la revendication 1, **caractérisé en ce que** pour la préparation d'un composé de formule (XVI) on fait réagir un composé de formule 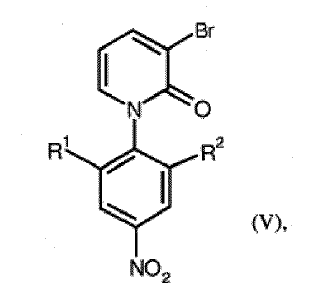 dans laquelle
R¹ représente un atome d'hydrogène ou un groupe alkyle en C₁-C₃
et
R² représente un groupe alkyle en C₁-C₃,
ou
R¹ représente un atome d'hydrogène
et
R² représente un groupe alcoxy en C₁-C₃ ou alcoxy(C₁-C₃)méthyle,
dans un procédé en un seul récipient, d'abord, en présence d'un catalyseur au cuivre, avec un ester d'acide malonique et une base et ensuite avec une base et de l'eau.

3. Procédé pour la préparation d'un composé de formule 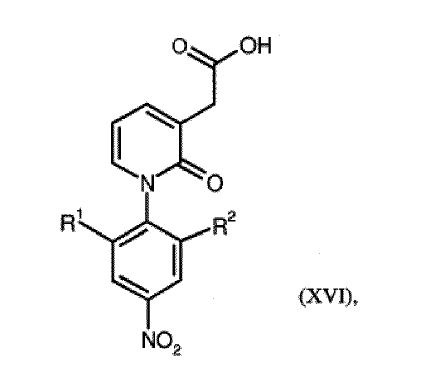 dans laquelle
R¹ représente un atome d'hydrogène ou un groupe alkyle en C₁-C₃
et
R² représente un groupe alkyle en C₁-C₃,
ou
R¹ représente un atome d'hydrogène
et
R² représente un groupe alcoxy en C₁-C₃ ou alcoxy(C₁-C₃)méthyle,
**caractérisé en ce qu'**on fait réagir un composé de formule 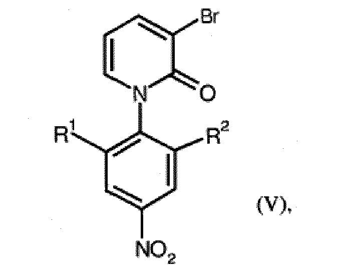 dans laquelle
R¹ représente un atome d'hydrogène ou un groupe alkyle en C₁-C₃
et
R² représente un groupe alkyle en C₁-C₃,
ou
R¹ représente un atome d'hydrogène
et
R² représente un groupe alcoxy en C₁-C₃ ou alcoxy(C₁-C₃)méthyle,
dans un procédé en un seul récipient, d'abord, en présence d'un catalyseur au cuivre, avec un ester d'acide malonique et une base et ensuite avec une base et de l'eau.

4. Procédé selon l'une quelconque des revendications 1, 2 ou 3, **caractérisé en ce que** dans un composé de formule (VII) R¹ représente le groupe méthyle et R² représente le groupe méthyle, dans un composé de formule (XVI) R¹ représente le groupe méthyle et R² représente le groupe méthyle et dans un composé de formule (V) R¹ représente le groupe méthyle et R² représente le groupe méthyle.

5. Procédé selon l'une quelconque des revendications 1, 2 ou 4, **caractérisé en ce que** le complexe de borane est le complexe borane-sulfure de diméthyle, borane-tétrahydrofurane, borane-diéthylamine ou catécholborane.

6. Procédé selon l'une quelconque des revendications 1, 2, 4 ou 5, **caractérisé en ce que** le complexe de borane est utilisé en excès.

7. Procédé selon l'une quelconque des revendications 2, 3 ou 4, **caractérisé en ce que** le catalyseur au cuivre est un complexe bis-(2-pyridinecarboxylato)cuivre(II) préparé au préalable ou ses diverses formes d'hydrates, ou a été préparé in situ à partir d'iodure de cuivre, de chlorure de cuivre, bromure de cuivre(II), triflate de cuivre(II) ou sulfate de cuivre(II), avec de l'acide 2-pyridine-carboxylique.

8. Procédé selon la revendication 7, **caractérisé en ce que** le catalyseur au cuivre est un complexe bis-(2-pyridinecarboxylato)cuivre(II) préparé au préalable ou ses diverses formes d'hydrates.

9. Procédé selon l'une quelconque des revendications 2, 3 ou 4, **caractérisé en ce que** l'ester d'acide malonique est le malonate de diéthyle, le malonate de dibenzyle, le malonate de di-tert-butyle ou le malonate d'éthyle et de potassium.

10. Procédé selon la revendication 9, **caractérisé en ce que** l'ester d'acide malonique est le malonate de diéthyle.

11. Procédé selon l'une quelconque des revendications 2, 3 ou 4, **caractérisé en ce que** la base est le tert-butylate de sodium ou de potassium, le carbonate de potassium, sodium ou césium, le fluorure de césium ou l'hydrure de sodium.

12. Procédé selon la revendication 11, **caractérisé en ce que** la base est le tert-butylate de sodium ou de potassium.

13. Composé de formule 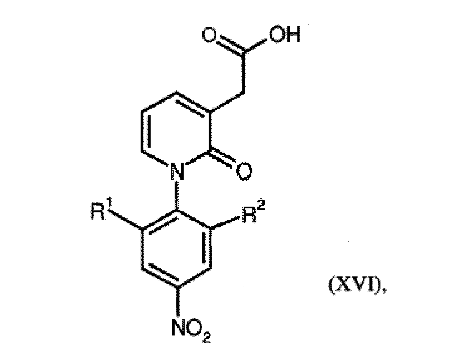 dans laquelle
R¹ représente un atome d'hydrogène ou un groupe alkyle en C₁-C₃
et
R² représente un groupe alkyle en C₁-C₃,
ou
R¹ représente un atome d'hydrogène
et
R² représente un groupe alcoxy en C₁-C₃ ou alcoxy(C₁-C₃)méthyle,
ou un de ses sels, de ses produits de solvatation ou des produits de solvatation de ses sels.

14. Composé selon la revendication 13, **caractérisé en ce que** R¹ représente le groupe méthyle et R² représente le groupe méthyle.
